# EUROPEAN PATENT SPECIFICATION

(11) **EP 2 963 260 B1**
(45) Date of publication and mention of the grant of the patent: **14.10.2020**
(21) Application number: 14743139.9
(22) Date of filing: 15.01.2014
(51) Int. Cl.: F01N 3/18, F01N 3/08, G01F 23/30, F01N 3/20, F01N 3/28, G01F 23/64, G01F 1/00, G01N 33/18, G01F 23/00

(54) **UREA WATER CONSUMPTION AMOUNT DIAGNOSTIC DEVICE FOR UREA SCR**
VORRICHTUNG ZUR DIAGNOSTIZIERUNG EINES HARNSTOFFWASSERVERBRAUCHS FÜR EINE SELEKTIVE KATALYTISCHE REDUKTION MIT HARNSTOFF
DISPOSITIF DE DIAGNOSTIC DE QUANTITÉ D'EAU/URÉE CONSOMMÉE POUR UNE RCS D'URÉE

(30) Priority: 28.01.2013 JP 2013013387
(43) Date of publication of application: 06.01.2016
(73) Proprietor: Isuzu Motors Limited, Tokyo 140-8722 (JP)
(72) Inventor: NIHONGI, Shigeru, Fujisawa-shi Kanagawa 252-0881 (JP); FUKUOKA, Takeshi, Fujisawa-shi Kanagawa 252-0881 (JP); MINEZAWA, Masanobu, Fujisawa-shi Kanagawa 252-0881 (JP)
(74) Representative: Schaumburg und Partner Patentanwälte mbB
(86) International application number: PCT/JP2014/050563
(87) International publication number: WO 2014/115619

(56) References cited:
- EP-A1- 2 071 145
- WO-A1-2012/144949
- JP-A- 2004 138 052
- JP-A- 2008 274 765
- JP-A- 2008 291 828
- JP-A- 2009 079 584
- JP-A- 2010 151 094
- JP-A- 2010 151 094

## Description

### TECHNICAL FIELD

The present invention relates to a urea SCR system for selectively reducing NOx contained in an exhaust gas of an engine with urea water, and more particularly to a urea water consumption diagnostic device for urea SCR that can accurately diagnose an amount of urea water sprayed (injected) from a dosing valve.

### BACKGROUND ART

An SCR system (selective catalytic reduction system) that uses a selective reduction catalyst is developed as an exhaust gas purification system to purify NOx contained in an exhaust gas of a diesel engine.

This SCR system supplies urea water, which is retained or pooled in a urea water tank, to an upstream exhaust gas of an SCR device to hydrolyze the urea water with the heat of the exhaust gas and generate ammonia. The SCR system then uses the ammonia to reduce NOx with a catalyst inside the SCR device, thereby purifying the exhaust gas. The urea water is sprayed from a dosing valve disposed upstream of the SCR device to supply the urea water to the upstream exhaust gas of the SCR device.

Feeding of the urea water to the dosing valve is carried out by a supply module that includes a supply module pump (SM pump), a urea water pressure sensor and other components. The supply module is connected to the urea water tank via a draw-in line (suction line), and supplies the urea water, which is drawn in from the urea water tank via the draw-in line, to the dosing valve through a pressurized urea water feed line extending between the supply module and the dosing valve. The dosing valve is controlled by a DCU (dosing control unit) such that opening and closing of the dosing valve is controlled in response to a detection value of a NOx sensor disposed downstream of the SCR device to adjust an amount of urea to be sprayed (urea spray volume).

A level (height or depth) of the urea water in the urea water tank is detected by a urea water sensor disposed in the urea water tank. A remaining amount of urea water is detected from the level of the urea water detected by the urea water sensor, and is used as a reference value for supplementation of the urea water.

### LISTING OF REFERENCES

### PATENT LITERATURES

PATENT LITERATURE 1: Japanese Patent Application Laid-Open Publication No. 2011-247137
PATENT LITERATURE 2: Japanese Patent Application Laid-Open Publication No. 2012-2061

Document EP 2 071 145 A1 discloses that in an exhaust emission purifying apparatus for reductively purifying NOx in the exhaust emission by using a liquid reducing agent or its precursor (to be referred to "liquid reducing agent" hereunder), a dosing flow rate of the liquid reducing agent according to engine operating conditions is computed, and the dosing of the liquid reducing agent is controlled based on the computed dosing flow rate, and also, it is judged whether or not a supply system of the liquid reducing agent is failed, based on a ratio between an integrated amount obtained by sequentially integrating the dosing flow rate and a consumed amount of the liquid reducing agent.

Document JP 2010151094 A discloses a reducer leak detection device and a leak detection method capable of detecting leak of a reducing agent in a storage tank. Document WO 2012/144949 A1 discloses a method and a device for the determination of a remaining volume of reducing agent in a container pertaining to an SCR system. A level measurement error due to splashing is compensated for a laps of time by setting a measurement of a remaining volume to a lower discrete indication.

### SUMMARY OF THE INVENTION

### PROBLEMS TO BE SOLVED BY THE INVENTION

When the dosing valve for spraying (injecting) the urea water into the NOx catalyst is clogged with a foreign matter and/or the pressurized urea water feed line extending to the dosing valve is clogged with a foreign matter, there is provided no means for detecting such clogging. Also, when the dosing valve is stuck in an open condition due to adhesion of a foreign matter or the like, there is a problem, i.e., there is provided no means for detecting such situation.

An amount (volume) of urea water consumption may be detected from a change in the detection level of the urea water sensor in the urea water tank. The detected amount of consumption is compared to a cumulative value of the sprayed urea water, which is injected from the dosing valve. If there is a substantial difference between the detected consumption amount and the cumulative value, then it is possible to detect (determine) that the dosing valve is stuck by a foreign matter or the like.

However, detection accuracy of the urea water sensor is designed to detect the urea level in a stepwise manner. Thus, the detection accuracy of the urea water sensor is bad. To deal with this, the inventors have proposed the following approach. A determination volume, for example 11-19 liters, is decided, such that the determination volume is equal to or greater than a detection error of the urea water sensor. When the cumulative sprayed volume from the dosing valve reaches the determination volume, the consumed volume is compared to the cumulative sprayed volume to diagnose the adhesion of a foreign matter and the like.

In this proposal, the detection accuracy of the urea water sensor is taken into account, and the diagnosis starts when the cumulative sprayed volume of the urea water exceeds the determination volume (e.g., 15 liters). However, if the urea water is frequently supplemented into the urea water tank before the diagnosis starts, then it is necessary to add the supplemented volume to the consumed volume, which is obtained from the level change, to calculate an actual volume of consumption.

As described above, however, the urea water sensor detects the level stepwise or digital-wise (e.g., in twenty steps). On the other hand, the liquid surface position (liquid level) of the urea water changes continuously or analog-wise. When a volume of supplementation that can change analog-wise is detected digital-wise, a level error of 2 steps may occur at most. For example, when the urea water supplementation is made immediately after the liquid surface position of the urea water is detected by the urea water sensor, when the urea water supplementation is made immediately before the liquid surface position of the urea water is detected by the urea water sensor, when the liquid level lifted upon supplementation does not reach the level to be detected by the urea water sensor, or when the liquid level lifted upon supplementation exceeds the level to be detected by the urea water sensor, a level error of 2 steps can occur at most. If this supplementation is repeated, a problem arises, i.e., the quantization error at the time of consumption volume determination increases.

Therefore, an object of the present invention is to overcome the above-described problems, and provide a urea water consumption diagnostic device for urea SCR which calculates an appropriate urea water consumption amount taking into account quantization errors of a urea water sensor due to supplementation (replenishment), and compares the calculated amount to a cumulative injected amount, which is injected from a dosing valve, to detect abnormality in the dosing valve and/or other elements.

### SOLUTIONS TO THE PROBLEMS

In order to achieve the above-mentioned object, a urea solution consumption diagnostic device for urea selective catalytic reduction system configured to suck water-based urea solution from a urea solution tank with a supply pump and spray the urea solution from a dosing valve disposed upstream of a selective catalytic reduction device via a pressurized urea solution feed line according to claim 1 is provided. The urea solution consumption diagnostic device of the present invention includes: a urea solution sensor configured to detect a level of the urea solution in the urea solution tank in a stepwise manner; a consumption volume calculating unit for calculating a cumulative consumption volume from a detection value entered from the urea solution sensor, and calculating a supplemented volume from the level change of the urea solution sensor upon supplementation of the urea water, to correct the cumulative consumption volume; and an abnormality diagnosing unit for, when the cumulative instructed spray volume of the urea solution injected from the dosing valve becomes equal to or greater than a determination volume, comparing the cumulative consumption volume calculated by the consumption volume calculating unit to the cumulative instructed spray volume to determine presence or absence of clogging of the urea solution in the pressurized urea solution feed line or the dosing valve. The consumption volume calculating unit subtracts one level from the changed level value of the urea solution sensor upon supplementation of the urea solution to the urea solution tank, and stores the resulting level value as the supplemented volume.

According to the invention, the consumption volume calculating unit subtracts one level from the changed level value of the urea solution sensor upon supplementation of the urea solution to the urea solution tank, and stores the resulting level value as the supplemented volume. Preferably, the consumption volume calculating unit adds the supplemented volume to the cumulative consumption volume, which is calculated from a value given from the urea solution sensor when the abnormality diagnosing unit performs the abnormality diagnosis, and sends the resulting value, as the cumulative consumption volume, to the abnormality diagnosing unit.

Preferably, when the abnormality diagnosing unit receives the cumulative instructed spray volume from an instructed spray volume addition unit and the cumulative consumption volume from the consumption volume calculating unit, and the cumulative instructed spray volume exceeds the determination volume, then the abnormality diagnosing unit compares the cumulative instructed spray volume to the cumulative consumption volume at this point in time. Preferably, if the difference between these two volumes exceeds a predetermined amount, the abnormality diagnosing unit determines that the urea solution consumption is abnormal. Preferably, if the difference between these two volumes does not exceed the predetermined amount, the abnormality diagnosing unit determines that the urea solution consumption is normal.

Preferably, the determination volume is set to a value between several liters and nineteen liters in terms of the cumulative instructed spray volume of the urea solution.

### ADVANTAGES OF THE INVENTION

In the present invention, when an instructed spray volume of the urea water is compared to a cumulative consumption volume variation derived from the level change of the urea water, the quantization error of supplemented volume due to the level change of the urea water sensor upon supplementation is minimized to obtain an appropriate cumulative consumption volume of the urea water. The present invention, therefore, demonstrates an excellent advantage that it is possible to detect defects and malfunctioning such as clogging of the dosing valve or the pressurized urea water feed line, and unintentionally continued spraying due to the dosing valve being stuck in the open condition.

### BRIEF DESCRIPTION OF THE DRAWINGS

Fig. 1 is a schematic view of an SCR system according to one embodiment of the present invention.
Fig. 2 is a diagram useful to describe cases where quantization errors occur due to the liquid level detection by the urea water sensor when the urea water is supplemented to the urea water tank in the embodiment of the present invention.
Fig. 3a and 3b represent a continuous flowchart illustrating consumption volume diagnosis of a urea water consumption diagnostic device for urea SRC according to the embodiment of the present invention.

### MODE FOR CARRYING OUT THE INVENTION

Now, the present invention will be described in detail with reference to the accompanying drawings.

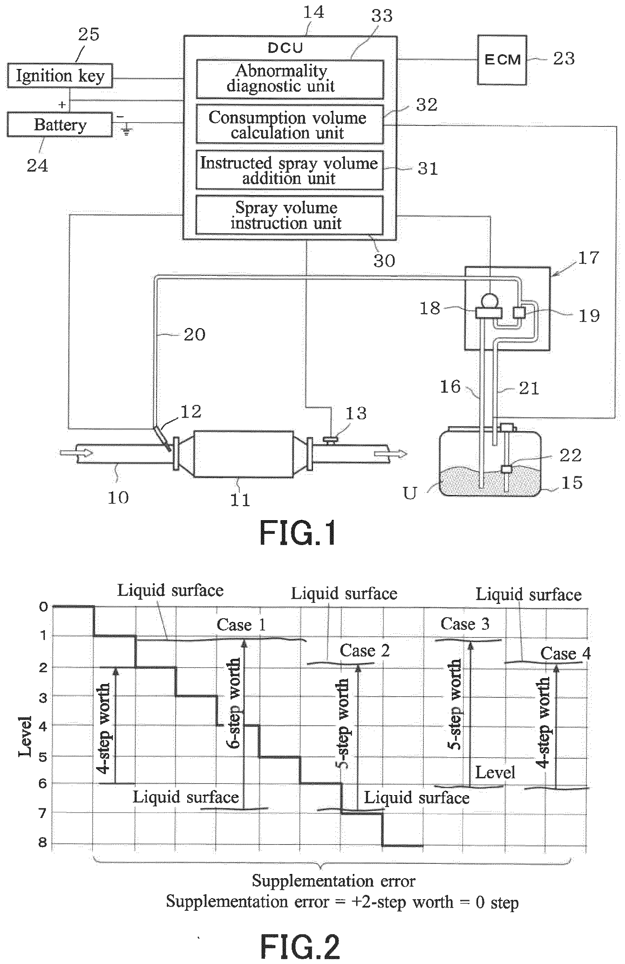

Fig. 1 illustrates a schematic configuration of an SCR system. An SCR device 11 is connected to an exhaust pipe 10 of a diesel engine (not shown), a dosing valve 12 is disposed upstream of the SCR device 11 for spraying (injecting) urea water, and a NOx sensor 13 is disposed downstream of the SCR device 11.

A detection value of the NOx sensor 13 is entered to a DCU (dosing control unit) 14, and closing and opening of the dosing valve 12 is controlled by the DCU 14.

Urea water U to be sprayed from the dosing valve 12 is retained (pooled) in a urea water tank 15. The urea water U is sucked in by a supply pump 18 of a supply module 17 via a suction line 16. Foreign matters are removed from the urea water by a filter 19, and the pressurized urea water U is sent to the dosing valve 12 from the supply pump 18 via a pressurized urea water feed line 20. Excessive urea water U is returned to the urea water tank 15 from the pressurized urea water feed line 20 via a return line 21 on the exit side of the filter 19.

A urea water sensor 22 is disposed in the urea water tank 15. The urea water sensor 22 measures a level of the urea water in the urea water tank 15, and sends the measured level to the DCU 14.

The DCU 14 calculates a volume (amount) of urea water to be sprayed to the SCR device 11 and timing of spraying the urea water. The DCU 14 drives the supply pump 18 to increase the pressure of the urea water to prescribed pressure, and controls the opening and closing of the dosing valve 12 to spray an appropriate volume of urea water at appropriate timing.

In order to monitor that the urea water is appropriately sprayed from the dosing valve 12 and therefore the NOx value in the exhaust gas downstream of the SCR device 11 has a stable value, the NOx sensor 13 sends a measured value to the DCU 14.

The DCU 14 is connected to an ECM (engine control module) 23, which is primarily provided for fuel injection control, and various input information such as a vehicle speed signal as well as control information of the ECM 23 is sent to the DCU 14 from the ECM 23.

The DCU 14 is also connected to a battery 24, and an ON signal and an OFF signal of an ignition key 25 are entered to the DCU 14.

In this SCR system, the DCU 14 decides an instruction for a volume of urea water to be sprayed (instructed spray volume) from the dosing valve 12 on the basis of the information of the ECM 23 such that a detection value of the NOx sensor 13 becomes stable (becomes a desired value). The DCU 14 controls the opening and closing of the dosing valve 12 on the basis of the decided volume. When clogging occurs in the passage to the dosing valve 12 from the supply pump 18 through the pressurized urea water feed line 20 due to foreign matters or the like, it is not possible to determine whether the urea water is appropriately sprayed from the dosing valve 12. Also, if the dosing valve 12 is stuck in the open condition and not closed, then the urea water is continuously sprayed from the dosing valve 12. This situation cannot be detected, either.

To deal with it, the DCU 14 of the present invention includes a spray volume instruction unit 30 for instructing a volume of urea water to be sprayed from the dosing valve 12 based on the information of the ECM 23 and the detection value of the NOx sensor 13, an instructed spray volume addition unit 31 for adding up the spray volume instructed by the spray volume instruction unit 30, a consumption volume calculation unit 32 for calculating a cumulative consumption volume D from the detection value entered from the urea water sensor 22, and an abnormality diagnostic unit 33 for comparing the cumulative instructed spray volume P from the instructed spray volume addition unit 31 to the cumulative consumption volume D from the consumption volume calculation unit 32 to determine whether the urea water spraying from the dosing valve 12 is normal or abnormal.

Firstly, when the diagnosis starts, the cumulative instructed spray volume P of the instructed spray volume addition unit 31 is reset to zero. At the same time, the consumption volume calculation unit 32 stores the level detected by the urea water sensor 22 as the level (S0). Subsequently, as the vehicle travels and the urea water is sprayed from the dosing valve 12, the instructed spray volume addition unit 31 successively adds up the instructed spray volume decided by the spray volume instruction unit 30, and stores the cumulative instructed spray volume P.

In order for the abnormality diagnostic unit 33 to detect whether the urea water spraying is normal or abnormal, a certain volume of urea water should be consumed (e.g., the consumed volume should be between several liters and nineteen liters) because otherwise the detection accuracy would not be high. Thus, when the vehicle's traveling is carried out several times, i.e., the ignition key 25 is turned on and off repeatedly, and the cumulative instructed spray volume P of the instructed spray volume addition unit 31 exceeds a determination volume L (e.g., 15 liters or 15L), then the consumption volume calculation unit 32 compares the cumulative consumption volume D to the cumulative instructed spray volume P to determine whether |D - P| > K1 is satisfied or not.

If this determination indicates that the urea water is appropriately sprayed, the cumulative consumption volume D is equal to the cumulative instructed spray volume P. When the difference between the cumulative consumption volume D and the cumulative instructed spray volume P is no greater than the value K1, which is decided on the basis of a level measurement tolerance (error range) of the urea water sensor 22, it is determined that the spraying is normal. If the difference is equal to or greater than the value K1, it is determined that the spraying is abnormal. In this determination, the absolute value of the difference between the two volumes is used for comparison. Thus, when there is no spraying due to a foreign matter or the like, D<<P is established and the value of D - P becomes minus. When the dosing valve 12 keeps spraying, D>>P is established and the value of D - P becomes plus. Accordingly, it is possible to determine from the value being plus or minus whether the clogging has occurred due to the stuck condition or the continuous spraying has occurred unintentionally from the dosing valve 12.

Because the urea water is supplemented to the urea water tank 15, the level indicated by the level sensor, which is the urea water sensor 22, is detected upon turning on and off the key switch, and the consumption volume calculation unit 32 decides a supplemented volume based on the change in the level indicated by the level sensor. Specifically, the consumption volume calculation unit 32 adds up the supplemented volume from the start of the control until the diagnosis is performed. Then, the consumption volume calculation unit 32 obtains a cumulative consumption volume D of the urea water by adding the cumulative supplemented volume to the actual level change.

However, the level detection by the urea water sensor 22 is carried out, for example, in twenty steps. When the urea water is frequently supplemented, the quantization error occurs.

This quantization error upon supplementation (supplementation error) will be described with reference to Fig. 2.

In Fig. 2, Level 0 indicates the level when the urea water is full (i.e., when the urea water surface is the highest) in the urea water tank. Fig. 2 shows that the level drops from Level 0 to Level 1, Level 2, ... and Level 8 as the liquid surface drops. The urea water sensor detects that the liquid surface level is Level 1 when the liquid surface drops to Level 1 from Level 0. Upon subsequent dropping of the liquid surface level, the urea water sensor detects that the liquid surface level becomes Level 2. Upon another subsequent dropping of the liquid surface level, the urea water sensor detects the liquid surface level becomes Level 3. On the other hand, when the urea water is supplemented, the urea water sensor detects that the liquid surface level is Level 7 when the liquid surface shifts to Level 7 from Level 8.

It should be assumed here that the urea water is supplemented by a volume equivalent to four steps (four-step worth of urea water is supplemented). If one step is equivalent to one liter in this case, the supplemented volume that corresponds to the four-step worth of level change is between four litters and five litters inclusive. Case 1 to Caser 4 are assumed depending upon the liquid surface position and the supplemented volume.

Specifically, in Case 1, the liquid surface level is Level 6 prior to the supplementation, and the liquid surface level changes from Level 6, 5, 4, 3 and 2 upon supplementation. This is a 6-step worth of change. In Case 2, the liquid surface level changes to Level 6, 5, 4, 3 and 2. This is a 5-step worth of change. In Case 3, the liquid surface level changes from Level 6 to Level 5, 4, 3 and 2. This is a 5-step worth of change. In Case 4, the liquid surface level changes to Level. 6, 5, 4, 3 and 2. This is a 4-step worth of change.

Therefore, the supplementation error is two-step worth at most in Case 1. In Case 2 and Case 3, the supplementation error is one step worth at most. In Case 4, the supplementation error is zero step.

Referring to Case 1 to Case 4, if the supplemented volume is four liters, which correspond to four-step worth of level, the supplementation error is zero step as in Case 4. If the supplemented volume is greater than four liters, the supplementation errors arise as in Case 1 to Case 3. In order to suppress the supplementation error (i.e., to reduce the absolute value), one step worth (R_{REF}) is subtracted from the supplemented volume which is obtained from the level change when the consumption volume calculating unit 32 determines the supplemented volume. This makes the supplementation error ± one step worth, and therefore the absolute value of the error is reduced to a half.

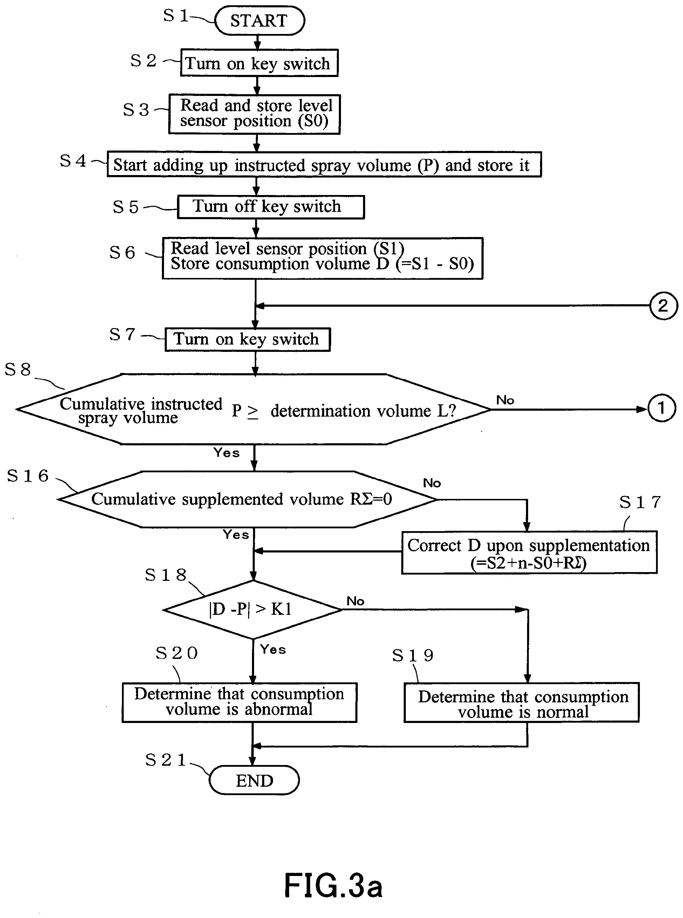

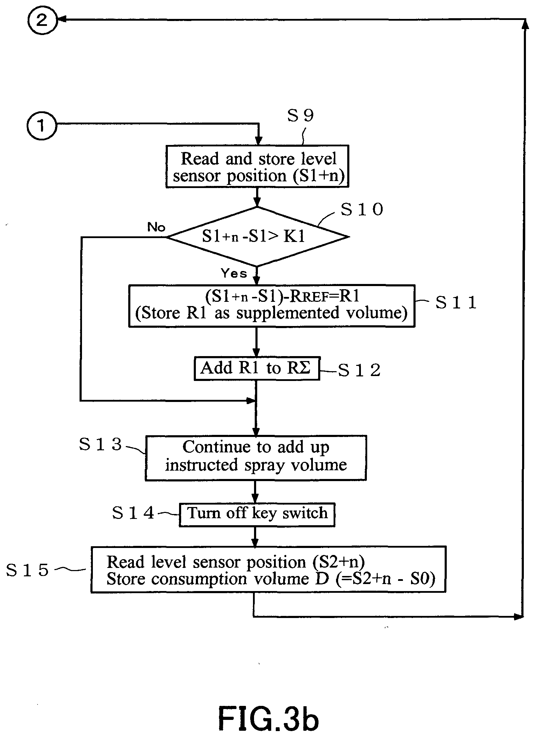

Now, the present invention will be described with reference to the flowchart of Fig. 3a and 3b.

At Step S1, the diagnosis starts. When the key switch is turned on (Step S2), the urea water sensor reads and stores the initial level sensor position (S0) (Step S3). Then, the instructed spray volume addition is started at Step S4. At Step S4, the instructed spray volume is added up until the key switch is turned off at Step S5, and the urea water level is stored as the level S0. When the key switch is turned off at Step S5, the level sensor position S1, which is the level sensor position after the first time traveling since the start of the control, is read, and the cumulative consumption volume D (= S1 - S0) of the urea water at that point in time is stored (Step S6).

When the key switch is turned on at Step S7, it is determined at Step S8 whether the cumulative instructed spray volume P of the previous traveling reaches the determination volume L (P≥L). The determination volume L is set to a value between several liters and nineteen liters, e.g., 15 liters.

If it is determined at Step S8 that the cumulative instructed spray volume P of the urea water does not reach the determination volume L (No), the control proceeds to Step S9 to read and store the level sensor position (S1+ₙ). Then, in the determination of Step S10, the stored level (S1₊₁) is compared to the level S1, which is the level prior to turning on of the key switch at Step S11, to determine whether S1₊ₙ - S1 > K1 is established or not. This determines whether there is any supplementation of the urea water. If there is no supplementation (No), the control proceeds to Step S13. If there is supplementation (Yes), a calculation of (S1₊ₙ - S1) - R_{REF} = R1 is made at Step S11. R1 is stored as the supplemented volume. Then, the supplemented volume R1 is added to the cumulative supplemented volume R∑ at Step S12, and the program proceeds to Step S13. At Step S13, the instructed spray volume addition is continued. After that, if the key switch is turned off at Step S14, the level sensor position (S2₊ₙ) is read at Step S15. The cumulative consumption volume D (= (S2₊ₙ - S1) - R_{REF}) is calculated based on this level (S2₊ₙ) and stored. Then, the program returns to the upstream of Step S7. Subsequently, when the key switch is turned on at Step S7, it is determined at Step S8 whether or not the cumulative instructed spray volume P of the urea water reaches the determination volume L. If the cumulative instructed spray volume P does not reach the determination volume L, the above-described Steps S9-S15 and S7 are repeated.

By repeating Steps S9-S15 and S7, a quantization error (R_{REF}) of the urea water sensor is subtracted beforehand (prior to Step S14). At Step S14, the cumulative consumption volume D (= (S2₊ₙ - S1) - R_{REF}) is calculated after supplementation. Therefore, it is possible to obtain an appropriate value of supplementation R1.

Subsequently, when it is determined at Step S8 that the cumulative instructed spray volume P reaches the determination volume L (Yes), it is then determined at Step S16 whether the cumulative supplemented volume R∑ is zero. If there is no supplementation (Yes), it is then determined at Step S18 whether |D - P|>K1 is established. If there is supplementation (No), the correction of the value D upon supplementation is made at Step S17 (D = S2₊₁ - S1 + R∑). Then, the program returns to carry out the determination at Step S18.

If it is determined at Step S18 that the spraying of the urea water from the dosing valve is normal, the cumulative consumption volume D is substantially equal to the cumulative instructed spray volume P, i.e., zero. It falls within the detection tolerance K1 (No). Accordingly, it is determined at Step S19 that the consumption volume is normal, and the control is finished (Step S21). If it is determined at Step S18 that the dosing valve or the like is clogged and no spraying is carried out, the cumulative consumption volume D is sufficiently smaller than the cumulative instructed spray volume P. If the dosing valve is stuck in the open condition such that the dosing valve keeps spraying the urea water, then the cumulative consumption volume D is sufficiently larger than the cumulative instructed spray volume P. Thus, the absolute value of the difference between the two volumes (|D - P|) is compared to the value K1. If the absolute value is greater than the value K1, it is determined at Step S20 that the consumption volume is abnormal, and the control is finished (Step S21).

### REFERENCE NUMERALS AND SYMBOLS

- 10:: Exhaust pipe
- 11:: SCR device
- 12:: Dosing valve
- 13:: NOx sensor
- 15:: Urea water tank
- 22:: Urea water sensor
- 30:: Spray volume instruction unit
- 31:: Instructed spray volume addition unit
- 32:: Consumption volume calculation unit
- 33:: Abnormality diagnostic unit

## Claims

1. A urea water consumption diagnostic device for a urea selective catalytic reduction system configured to suck urea water from a urea water tank (15) with a supply pump (18) and spray the urea water from a dosing valve (12) disposed upstream of a selective catalytic reduction device (11) via a pressurized urea water feed line (20), the device comprising:
a urea water sensor (22) configured to detect a level of the urea water in the urea water tank (15) ;
consumption volume calculating means (32) configured to calculate a cumulative consumption volume from a detection value entered from the urea water sensor (22), and calculate a supplemented volume from a change in the level of the urea water sensor (22) upon supplementation of the urea water, thereby correcting the cumulative consumption volume; and
abnormality diagnosing means (33) configured to, when a cumulative instructed spray volume of the urea water sprayed from the dosing valve (12) becomes equal to or greater than a determination volume, compare the cumulative consumption volume calculated by the consumption volume calculating means (32) to the cumulative instructed spray volume to detect defects and malfunctioning such as clogging of the dosing valve (12) or the pressurized urea water feed line (20), and unintentionally continued spraying due to the dosing valve (12) being stuck in the open condition,
**characterised by** the urea water sensor (22) being configured to detect a level of urea water in the urea tank (15) in a stepwise manner;
the consumption volume calculating means (32) being configured to:
store level values of the urea water sensor (22) when a key switch is turned off and on;
calculate a changed level value (S1₊ₙ-S1) which is a difference between a level value (S1₊ₙ) stored when the key switch is turned on and a level value (S1) stored when the key switch is turned off and before the key switch is turned on; and
when the changed level value (S1₊ₙ-S1) is greater than a predetermined value (K1), determine that the urea water is supplemented to the urea water tank (15), subtract one level (R_{REF}) from the changed level value, and store a resulting level value (R1) as the supplemented volume.

2. The urea water consumption diagnostic device for a urea selective catalytic reduction system according to claim 1, wherein the consumption volume calculating means (32) is configured to subtract one level from the changed level value of the urea water sensor (22) upon supplementation of the urea water to the urea water tank (15), and store the resulting level value as the supplemented volume, and the consumption volume calculating means (32) is configured to add the supplemented volume to the cumulative consumption volume, which is calculated from the urea water sensor (22), when the abnormality diagnosing means (33) performs abnormality diagnosis, and sends a resulting value, as the cumulative consumption volume, to the abnormality diagnosing means (33).

3. The urea water consumption diagnostic device for a urea selective catalytic reduction system according to claim 1 or 2, wherein when the abnormality diagnosing means (33) receives the cumulative instructed spray volume from instructed spray volume addition means (31) and the cumulative consumption volume from the consumption volume calculating means (32), and the cumulative instructed spray volume exceeds the determination volume, then the abnormality diagnosing means (33) compares the cumulative instructed spray volume to the cumulative consumption volume at this point in time, and if a difference between the cumulative instructed spray volume and the cumulative consumption volume exceeds a predetermined volume, the abnormality diagnosing means (33) determines that urea water consumption is abnormal whereas if the difference between the cumulative instructed spray volume and the cumulative consumption volume does not exceed the predetermined volume, the abnormality diagnosing means (33) determines that the urea water consumption is normal.

4. The urea water consumption diagnostic device for a urea selective catalytic reduction system according to any one of claims 1 to 3, wherein the determination volume is set to a value between several liters and nineteen liters in terms of the cumulative instructed spray volume of the urea water.

## Patentansprüche

1. Harnstoff-Wasser-Verbrauchsdiagnosevorrichtung für ein selektives katalytisches Reduktionssystem mit Harnstoff, die ausgebildet ist, Harnstoff-Wasser aus einem Harnstoff-Wasser-Tank (15) mit einer Zuführpumpe (18) zu saugen und das Harnstoff-Wasser über eine unter Druck gesetzte Harnstoff-Wasser-Zuführleitung (20) aus einem Dosierventil (12), das stromaufwärts einer selektiven katalytischen Reduktionsvorrichtung (11) angeordnet ist, einzuspritzen, wobei die Vorrichtung umfasst:
einen Harnstoff-Wasser-Sensor (22), der ausgebildet ist, um einen Pegel des Harnstoff-Wassers in dem Harnstoff-Wasser-Tank (15) zu erfassen;
Verbrauchsvolumenberechnungsmittel (32), die ausgebildet sind, um ein kumulatives Verbrauchsvolumen anhand eines Erfassungswerts zu berechnen, der aus dem Harnstoff-Wasser-Sensor (22) eingegeben wird, und ein ergänztes Volumen anhand einer Veränderung des Pegels des Harnstoff-Wasser-Sensors (22) bei Ergänzung des Harnstoff-Wassers zu berechnen und dadurch das kumulative Verbrauchsvolumen zu korrigieren; und
Anomaliediagnosemittel (33), die ausgebildet sind, um, wenn ein kumulatives angewiesenes Einspritzvolumen des Harnstoff-Wassers, das aus dem Dosierventil (12) eingespritzt wird, gleich oder größer als ein Bestimmungsvolumen ist, das kumulative Verbrauchsvolumen, das von den Verbrauchsvolumenberechnungsmitteln (32) berechnet wird, mit dem kumulativen angewiesenen Einspritzvolumen zu vergleichen, um Fehler und Fehlfunktionen wie z. B. ein Verstopfen des Dosierventils (12) oder der unter Druck gesetzten Harnstoff-Wasser-Zuführleitung (20) und ein unbeabsichtigtes fortgesetztes Einspritzen aufgrund eines Feststeckens des Dosierventils (12) in dem geöffneten Zustand zu erfassen,
**dadurch gekennzeichnet, dass** der Harnstoff-Wasser-Sensor (22) ausgebildet ist, um einen Pegel des Harnstoff-Wassers in dem Harnstoff-Tank (15) schrittweise zu erfassen;
wobei die Verbrauchsvolumenberechnungsmittel (32) ausgebildet sind, um:
Pegelwerte des Harnstoff-Wasser-Sensors (22) zu speichern, wenn ein Schlüsselschalter aus- und eingeschaltet wird;
einen geänderten Pegelwert (S1₊ₙ-S1) zu berechnen, der eine Differenz zwischen einem Pegelwert (S1₊ₙ), der gespeichert wird, wenn der Schlüsselschalter eingeschaltet ist, und einem Pegelwert (S1) ist, der gespeichert wird, wenn der Schlüsselschalter ausgeschaltet ist und bevor der Schlüsselschalter eingeschaltet ist; und
wenn der geänderte Pegelwert (S1₊ₙ-S1) größer als ein vorbestimmter Wert (K1) ist, zu bestimmen, dass das Harnstoff-Wasser in dem Harnstoff-Wasser-Tank (15) ergänzt ist, einen Pegel (R_{REF}) von dem geänderten Pegelwert abzuziehen und einen resultierenden Pegelwert (P1) als ergänztes Volumen zu speichern.

2. Harnstoff-Wasser-Verbrauchsdiagnosevorrichtung für ein selektives katalytisches Reduktionssystem mit Harnstoff nach Anspruch 1, wobei die Verbrauchsvolumenberechnungsmittel (32) ausgebildet sind, um einen Pegel von dem geänderten Pegelwert des Harnstoff-Wasser-Sensors (22) bei Ergänzung des Harnstoff-Wassers zu dem Harnstoff-Wasser-Tank (15) abzuziehen und den resultierenden Pegelwert als ergänztes Volumen zu speichern, und die Verbrauchsvolumenberechnungsmittel (32) ausgebildet sind, das ergänzte Volumen zu dem kumulativen Verbrauchsvolumen zu addieren, das aus dem Harnstoff-Wasser-Sensor (22) berechnet wird, wenn die Anomaliediagnosemittel (33) eine Anomaliediagnose durchführen, und einen resultierenden Wert als kumulatives Verbrauchsvolumen an die Anomaliediagnosemittel (33) senden.

3. Harnstoff-Wasser-Verbrauchsdiagnosevorrichtung für ein selektives katalytisches Reduktionssystem mit Harnstoff nach Anspruch 1 oder 2, wobei, wenn die Anomaliediagnosemittel (33) das kumulative angewiesene Einspritzvolumen aus den Additionsmitteln (31) für das angewiesene Einspritzvolumen und das kumulative Verbrauchsvolumen aus den Verbrauchsvolumenberechnungsmitteln (32) empfangen und das kumulative angewiesene Einspritzvolumen das Bestimmungsvolumen übersteigt, dann die Anomaliediagnosemittel (33) das kumulative angewiesene Einspritzvolumen mit dem kumulativen Verbrauchsvolumen zu diesem Zeitpunkt vergleichen, und wenn eine Differenz zwischen dem kumulativen angewiesenen Einspritzvolumen und dem kumulativen Verbrauchsvolumen ein vorbestimmtes Volumen übersteigt, die Anomaliediagnosemittel (33) bestimmen, dass der Harnstoff-Wasser-Verbrauch abnormal ist, wohingegen, wenn die Differenz zwischen dem kumulativen angewiesenen Einspritzvolumen und dem kumulativen Verbrauchsvolumen das vorbestimmte Volumen nicht übersteigt, die Anomaliediagnosemittel (33) bestimmen, dass der Harnstoff-Wasser-Verbrauch normal ist.

4. Harnstoff-Wasser-Verbrauchsdiagnosevorrichtung für ein selektives katalytisches Reduktionssystem mit Harnstoff nach einem der Ansprüche 1 bis 3, wobei das Bestimmungsvolumen auf einen Wert zwischen mehreren Litern und neunzehn Litern in Bezug auf das kumulative angewiesene Einspritzvolumen des Harnstoff-Wassers eingestellt ist.

## Revendications

1. Dispositif de diagnostic de consommation d'eau d'urée pour un système de réduction catalytique sélective d'urée configuré pour aspirer de l'eau d'urée à partir d'un réservoir d'eau d'urée (15) à l'aide d'une pompe d'alimentation (18) et pulvériser l'eau d'urée à partir d'une vanne de dosage (12) disposée en amont d'un dispositif de réduction catalytique sélective (11) via une conduite d'alimentation en eau d'urée sous pression d'(20), le dispositif comprenant :
un capteur d'eau d'urée (22) configuré pour détecter un niveau de l'eau d'urée dans le réservoir d'eau d'urée (15) ;
des moyens de calcul de volume de consommation (32) configurés pour calculer un volume de consommation cumulé à partir d'une valeur de détection entrée à partir du capteur d'eau d'urée (22), et calculer un volume ajouté à partir d'un changement du niveau du capteur d'eau d'urée (22) lors de l'ajout de l'eau d'urée, en corrigeant ainsi le volume de consommation cumulé ; et
des moyens de diagnostic d'anomalie (33) configurés pour, lorsqu'un volume de pulvérisation d'instruction cumulé de l'eau d'urée pulvérisée depuis la vanne de dosage (12) devient égal ou supérieur à un volume de détermination, comparer le volume de consommation cumulé calculé par les moyens de calcul de volume de consommation (32) au volume de pulvérisation d'instruction cumulé pour détecter des défauts et des dysfonctionnements tels qu'un colmatage de la vanne de dosage (12) ou de la conduite d'alimentation en eau sous pression (20), et une pulvérisation continue non voulue du fait que la vanne de dosage (12) est coincée dans l'état ouvert,
**caractérisé par** le capteur d'eau d'urée (22) configuré pour détecter un niveau de l'eau d'urée dans le réservoir d'eau d'urée (15) de manière étagée ;
les moyens de calcul de volume de consommation (32) étant configurés pour :
stocker des valeurs de niveau du capteur d'eau d'urée (22) lorsqu'un commutateur à clé est désactivé et activé ;
calculer une valeur de niveau modifiée (S1_{+ n}-S1) qui est une différence entre une valeur de niveau (S1_{+ n}) stocker lorsque le commutateur à clé est activé et une valeur de niveau (S1) stockée lorsque le commutateur à clé est désactivé et avant que le commutateur à clé ne soit activé ; et
lorsque la valeur de niveau modifiée (S1_{+ n}-S1) est supérieure à une valeur prédéterminée (K1), déterminer que l'eau d'urée est ajoutée au réservoir d'eau d'urée (15), soustraire un niveau (R_{REF}) à partir de la valeur de niveau modifiée, et stocker une valeur de niveau résultante (R1) en tant que volume ajouté.

2. Dispositif de diagnostic de consommation d'eau d'urée pour un système de réduction catalytique sélective d'urée selon la revendication 1, dans lequel les moyens de calcul de volume de consommation (32) sont configurés pour soustraire un niveau à partir de la valeur de niveau modifiée du capteur d'eau d'urée (22) lors d'un ajout de l'eau d'urée au réservoir d'eau d'urée (15), et stocker la valeur de niveau résultante en tant que volume ajouté, et les moyens de calcul de volume de consommation (32) sont configurés pour ajouter le volume ajouté au volume de consommation cumulé, qui est calculé à partir du capteur d'eau d'urée (22), lorsque les moyens de diagnostic d'anomalie (33) effectuent un diagnostic d'anomalie et envoient une valeur résultante, en tant que volume de consommation cumulé, aux moyens de diagnostic d'anomalie (33).

3. Dispositif de diagnostic de consommation d'eau d'urée pour un système de réduction catalytique sélective d'urée selon la revendication 1 ou 2, dans lequel lorsque les moyens de diagnostic d'anomalie (33) reçoivent le volume de pulvérisation d'instruction cumulé par les moyens d'ajout de volume de pulvérisation d'instruction (31) et le volume de consommation cumulé à partir des moyens de calcul du volume de consommation (32), et le volume de pulvérisation d'instruction cumulé dépasse le volume de détermination, alors les moyens de diagnostic d'anomalie (33) comparent le volume de pulvérisation d'instruction cumulé au volume de consommation cumulé à cet instant, et si une différence entre le volume de pulvérisation d'instruction cumulé et le volume de consommation cumulé dépassent un volume prédéterminé, les moyens de diagnostic d'anomalie (33) déterminent que la consommation d'eau d'urée est anormale alors que si la différence entre le volume de pulvérisation d'instruction cumulé et le volume de consommation cumulé ne dépassent pas le volume prédéterminé, les moyens de diagnostic d'anomalie (33) déterminent que la consommation d'eau d'urée est normale.

4. Dispositif de diagnostic de consommation d'eau d'urée pour un système de réduction catalytique sélective d'urée selon l'une quelconque des revendications 1 à 3, dans lequel le volume de détermination est réglé sur une valeur entre plusieurs litres et dix-neuf litres en termes du volume de pulvérisation d'instruction cumulé de l'eau d'urée.
